# EUROPEAN PATENT APPLICATION

(11) **EP 4 722 382 A1**
(43) Date of publication of application: **08.04.2026**
(21) Application number: 24218499.2
(22) Date of filing: 09.12.2024
(51) Int. Cl.: C12Q 1/6816, C12Q 1/6806, C12N 15/10

(54) **PURIFYING HYBRIDIZED RNA POLYNUCLEOTIDES**

(30) Priority: 02.10.2024 US 202418904862
(71) Applicant: mbiomics GmbH, 82061 Neuried (DE)
(72) Inventor: Grabmayr, Heinrich E. P., 80803 Munich (DE); Grohme, Markus, 81377 Munich (DE); Goetz, Alexandra, 81825 Munich (DE); Ross, Breyan, 80687 Munich (DE); Woehrstein, Johannes B., 85354 Hohenbachern (DE)
(74) Representative: Stellbrink & Partner Patentanwälte mbB

(57) **Abstract**

A method of purifying hybridized RNA polynucleotides involves a washing step in which no ethanol or less than 5% ethanol is used in the buffer that washes the beads to which the hybridized RNA polynucleotides have precipitated so as to separate them from unbound DNA oligonucleotides. A binding buffer that includes the beads is combined with a hybridization solution that includes salt and RNA polynucleotides of bacterial species that are hybridized to DNA oligonucleotides. The binding buffer and the hybridization solution are removed from the aggregated beads. The washing buffer, as is conventionally used, is added to the beads after the binding buffer and the hybridization solution have been removed. The washing buffer is removed from the aggregated beads. An elution buffer is added to the beads after the washing buffer has been removed. The elution buffer which contains the purified hybridized RNA polynucleotides is removed from the aggregated beads.

## Description

### TECHNICAL FIELD

The present invention relates to the field of microbiome analysis and particularly to a method for purifying RNA polynucleotides hybridized to DNA oligonucleotides using beads and a washing buffer that does not separate the hybridized oligonucleotides from the polynucleotides. The purified, hybridized RNA polynucleotides can then be used to generate a quantitative fingerprint of a subset of bacteria in a person's microbiome.

### BACKGROUND INFORMATION

Microbiomes inside the human gastrointestinal tract play an important role in the immune system, metabolism and general health. The identity and abundance of the various species of bacteria in the human microbiome can be correlated to a number of conditions and diseases, such as cardiovascular disease, hypertension, cancer, diabetes, obesity and auto-immune diseases. In addition, the composition of bacteria in the gastrointestinal microbiome may also be a proxy for the probability of adverse reactions to medical treatments. Thus, various conditions and diseases can be diagnosed and various therapies prescribed by identifying and quantifying the relative presence of specific bacteria in a person's microbiome. One way of identifying and qualifying bacteria relies on the 16S component of ribosomal RNA. The 16S rRNA genes of most bacteria in the human gastrointestinal tract have been sequenced and are listed in publicly accessible databases, such as the GenBank of the National Institute of Health. One method of identifying the various bacteria in a patient's fecal (stool) sample involves analyzing the 16S rRNA of the bacteria using quantitative polymerase chain reaction (qPCR) analysis. However, qPCR amplification can be time consuming, inaccurate and a poor measure of the relative abundance of the various bacteria that are identified.

Another method of identifying the various bacteria species involves shotgun metagenomic sequencing and is based on sequencing the total extracted DNA of the gastrointestinal microbiome, which is bioinformatically decomposed into the known genomes of the potential bacteria. The benefits compared to 16S analysis are stain-level identification, high sensitivity, and the possibility to use the genetic information for further analysis. However, because of the highly complex reconstruction of the microbiome composition, shotgun metagenomic sequencing provides poor quantification.

One problem with current microbiome analysis methods is the limited range of the various concentrations that can be measured. The concentrations of the various types of bacteria in the human gastrointestinal tract can vary by orders of magnitude. The accuracy of current methods for quantifying multiple bacteria species in a single sample is lower when the concentrations of the bacteria species vary by orders of magnitude. For example, *Bacteroides vulgatus, Bacteroides uniformis,* and *Alistipes putredinis* are often present in human stool samples at a population size of 1-10%, while *Bacteroides fragilis* and *Bacteroides coprophilus* are present at a population size of 0.01%-0.1%, and *Abiotrophia defective* and *Acidaminococcus fermentans* occur at a population size of 0.00001%-0.0001%. Current analysis methods cannot accurately quantify the relative concentrations of these bacteria species in a single sample.

A method is sought that allows multiple target species of bacteria in a microbiomic sample to be accurately quantified over a wide range of concentrations of the target species. Accurately quantifying species of bacteria involves purifying RNA polynucleotides that have hybridized to DNA oligonucleotide binders, which can be used to immobilize and count the individually identifiable RNA polynucleotide subunits. Possible purification methods include (1) filtration using molecular weight cut-off (MWCO) membranes, (2) filtration using spin columns and chromatography resins, (3) extraction using Agarose gel electrophoresis to separate slow-migrating RNA polynucleotides as a distinct band from faster migrating oligonucleotides and then excising the desired band from the gel, (4) using polyethylene glycol (PEG) to induce precipitation of RNA, and (5) solid-phase reversible immobilization (SPRI) bead purification. All of these conventional purification techniques have limitations that prevent them from being used to achieve high yields of purified hybridized RNA polynucleotides. A method is sought for obtaining high yields of purified RNA polynucleotides hybridized to DNA oligonucleotides.

### SUMMARY

A method of purifying hybridized RNA polynucleotides is used in a method for quantifying bacteria which is defined in claim 1. The method of purifying hybridized RNA polynucleotides involves a washing step in which no ethanol is used in the buffer that washes the beads to which the hybridized RNA polynucleotides have precipitated so as to separate them from unbound DNA oligonucleotides. A binding buffer that includes beads is first combined with a hybridization solution that includes salt and the hybridized RNA polynucleotides that are hybridized to DNA oligonucleotides. The beads are then aggregated using a magnetic field. The binding buffer and the hybridization solution are removed from the aggregated beads. The washing buffer which does not include ethanol, as is conventionally used, is added to the beads after the binding buffer and the hybridization solution have been removed. Then the beads to which the washing buffer has been added are aggregated, and the washing buffer is removed from the aggregated beads. An elution buffer is added to the beads after the washing buffer has been removed. Then the beads to which the elution buffer has been added are aggregated. Finally, the elution buffer is removed from the aggregated beads. The hybridized RNA polynucleotides have been purified because the elution buffer contains a concentration of DNA oligonucleotides that are not hybridized to RNA polynucleotides that is lower than that in the hybridization solution

In another embodiment of the method of purifying hybridized RNA polynucleotides which is defined in claim 11, the washing step uses a washing buffer that includes less than 5% ethanol to wash the beads to which the hybridized RNA polynucleotides have precipitated so as to separate them from unbound DNA oligonucleotides.

In yet another embodiment, a method of purifying hybridized RNA polynucleotides in a sample is defined in claim 15 and involves adding a washing buffer to the sample and removing at least some of the washing buffer from the sample. The sample includes beads to which the hybridized RNA polynucleotides are bound and DNA oligonucleotides that are not hybridized to RNA polynucleotides. The washing buffer includes ethanol at a concentration of 5% or less. The method involves removing from the sample at least some of the washing buffer and at least some of the DNA oligonucleotides that are not hybridized to RNA polynucleotides.

The purification method of the invention is used in a method for determining a quantitative fingerprint of a predetermined subset of bacterial species in a gastrointestinal tract sample of a patient. The method for quantifying bacteria uses small DNA nanostructures to detect and count single nucleic acid molecules of the various target bacterial species. The bacteria quantifying method involves attaching a DNA nanostructure that fluoresces a predetermined fluorophore color to a ribosomal RNA (rRNA) polynucleotide subunit of the target bacterial species that corresponds to the predetermined color. The relative concentrations of the various bacterial species in the predetermined subset are determined based on how many DNA nanostructures with the predetermined fluorophore color for each target bacterial species are counted on the surface of a slide or microscopy chamber. The accuracy of the count of particular RNA polynucleotide subunits attached to fluorescing nanostructures depends on the purification of the RNA polynucleotides hybridized to DNA oligonucleotide binders. Free-floating DNA oligonucleotide binders must be removed from the annealing reaction solution without separating the two DNA oligonucleotide binders that are hybridized to each RNA polynucleotide subunit. The bead purification method of the invention involves a stringency-controlled washing buffer that keeps the desired hybridization between the RNA polynucleotides and the DNA oligonucleotides intact while washing away the unbound DNA oligonucleotides. If the hybridization between the RNA polynucleotides and the DNA oligonucleotides is not retained during the washing step, then the relative concentrations of the 16S rRNA polynucleotides of the various bacteria species, strains and other taxa (collectively called "species") in the sample cannot be accurately quantified.

After the hybridized RNA polynucleotides are purified and the DNA oligonucleotide binders are attached to fluorescing DNA nanostructures corresponding to each bacterial species, a quantitative fingerprint of bacterial abundance in the patient's gastrointestinal tract is determined. The relative concentration of each bacterial species is determined based on how many corresponding DNA nanostructures are counted on the surface of the slide or microscopy chamber. The quantitative fingerprint of bacterial abundance is then compared with the known relative abundance indicative of a particular disease or condition, and a therapy is administered to decrease the relative concentration of a particular strain of bacteria indicated by the quantitative fingerprint to be in overabundance in a patient's gastrointestinal tract compared to the bacterial concentrations in a healthy person. Without first performing the bead purification method of the invention, however, the quantitative fingerprint of relative concentrations of bacterial species is less accurate, and the administered therapy is less effective.

In another embodiment of a method of purifying hybridized RNA polynucleotides, the stringency of the washing buffer is adjusted to provide a melting temperature of 5 to 10 degrees Celsius above the temperature of the washing buffer as defined in claim 21. In a first step, a hybridization solution is added to a binding buffer and beads. The hybridization solution includes the hybridized RNA polynucleotides that are hybridized to DNA oligonucleotides. A first binding site with a first nucleotide sequence on the RNA polynucleotides is hybridized to a second binding site with a second nucleotide sequence on the DNA oligonucleotides. The beads are then aggregated using a magnetic field. The binding buffer and the hybridization solution are removed from the aggregated beads. A washing buffer is added to the beads after the binding buffer and the hybridization solution have been removed. The stringency of the washing buffer is adjusted to provide a melting temperature between the first binding site with the first nucleotide sequence and the second binding site with the second nucleotide sequence of 5 to 10 degrees Celsius above the temperature of the washing buffer. The adjustment of the stringency of buffers can be performed by adjusting the concentration of cosmotropic and chaotropic reagents, such as salts, e.g., NaCl or MgCl, or other reagents such as formamide or DMSO (dimethyl sulfoxide), which alter the melting temperature. The required concentrations for reaching a melting temperature can be approximated by calculation or by testing experimentally by melting curve analysis, e.g., by using intercalating dyes such as SYBR Green in a qPCR system like the LightCycler. The beads to which the washing buffer has been added are aggregated. The washing buffer is removed from the aggregated beads. An elution buffer is added to the beads after the washing buffer has been removed. The beads to which the elution buffer has been added are aggregated. The elution buffer is removed from the aggregated beads. The elution buffer contains purified hybridized RNA polynucleotides from which substantially all DNA oligonucleotides that are not hybridized to RNA polynucleotides have been removed.

Preferred embodiments of the method of the invention are defined in the subclaims.

Further details and embodiments and methods are described in the detailed description below. This summary does not purport to define the invention. The invention is defined by the claims.

### BRIEF DESCRIPTION OF THE DRAWINGS

The accompanying drawings, where like numerals indicate like components, illustrate embodiments of the invention.
FIG. 1 is a schematic diagram of a 16S ribosomal RNA subunit of a bacteria hybridized to an immobilizing binder and a fluorophore binder.
FIG. 2 shows a flowchart of steps of a novel method for purifying hybridized polynucleotides.
FIG. 3 illustrates a binding buffer including SPRI beads being combined with a hybridization solution in an annealing tube, such as a PCR tube.
FIG. 4 shows the PCR tube containing the combined bead suspension being placed on a magnetic rack, where the beads are aggregated into a pellet.
FIG. 5 shows a washing buffer being added to the beads and the remnants of the hybridization solution and binding buffer on the surface of the beads.
FIG. 6 shows the PCR tube containing the beads and washing buffer being placed on the magnetic rack, where the beads are aggregated into a pellet.
FIG. 7 shows a suspension in the PCR tube in which some of the hybridized RNA polynucleotides bound to the beads are hybridized to weaker bound oligonucleotides.
FIG. 8 shows an elution buffer being added to the beads after the washing buffer has been removed.
FIG. 9 shows the supernatant, which is the elution buffer containing the purified hybridized RNA polynucleotides, being removed from the aggregated beads.
FIG. 10 is a schematic diagram of a 16S ribosomal RNA subunit attached via a hybridized fluorophore binder to a fluorescent DNA nanostructure and via a hybridized immobilizing binder to a surface of a slide.
FIG. 11 is a perspective schematic diagram of an exemplary fluorescent DNA nanostructure used for quantifying bacteria.
FIG. 12 is a top view of the DNA nanostructure shown in FIG. 11.
FIG. 13 shows more detail of how organic fluorophores are attached to the DNA nanostructure of FIG. 11.
FIG. 14 is a table of predetermined species of bacteria as well as their relative abundance in a gastrointestinal microbiomic sample from a patient.

### DETAILED DESCRIPTION

Reference will now be made in detail to some embodiments of the invention, examples of which are illustrated in the accompanying drawings.

The method of the invention is used to purify hybridized polymers of ribonucleotides and deoxyribonucleotides, such as ribonucleic acid (RNA) obtained from subcellular organelles such as ribosomes. In the process of determining a quantitative fingerprint of relative bacterial concentrations, ribosomal RNA (rRNA) polynucleotide subunits that are attached by hybridization to smaller DNA oligonucleotides are purified using the method of the invention. The oligonucleotides referred to in this disclosure can include up to sixty nucleotides. In a preferred embodiment, the method involves adding a washing buffer to a sample comprising solid-phase reversible immobilization (SPRI) paramagnetic beads to which hybridized RNA polynucleotides are bound as well as DNA oligonucleotides that are not hybridized to RNA polynucleotides. Conventional SPRI bead purification requires the beads to be washed with a buffer that includes at least 65% ethanol. The inventors observed that purifying hybridized RNA polynucleotides was not successful in a process of quantifying bacterial species because the yield of the purified hybridized RNA polynucleotides was too low. The inventors recognized that the problem with conventional SPRI bead purification used to purify hybridized polynucleotides was that the high ethanol concentration was unbinding the hybridization and lowering the yield of hybridized RNA polynucleotides that were precipitating onto the beads. The solution was to purify the hybridized RNA polynucleotides without using ethanol or by using a low concentration of ethanol, such as 5% or less. The DNA oligonucleotides that are not hybridized to RNA polynucleotides are removed from the sample of hybridized RNA polynucleotides together with the washing buffer that includes only a low concentration, if any, of ethanol.

The method of purifying hybridized polynucleotides of the invention is applied after constructing the hybridized RNA polynucleotides, preferably 16S rRNA polynucleotide subunits, which are used together with fluorescent DNA nanostructures to determine a quantitative fingerprint of bacterial species in a microbiomic sample. This disclosure first generally describes the process of determining the quantitative fingerprint of bacterial species. In a preferred embodiment of the method, the sample is a gastrointestinal microbiomic sample.

A multiplexed nucleic acid detection assay is used to detect and count single target nucleic acid molecules of the various bacterial species in the gastrointestinal microbiomic sample. Fluorescent deoxyribonucleic acid (DNA)-based nanostructures are used to detect and quantify 16S rRNA molecules of the bacteria at the single molecule level. The target nucleic acid molecules of the bacteria are immobilized on a surface of a slide or microscopy chamber. A fluorescent DNA nanostructure is then hybridized with an immobilized target nucleic acid molecule. Distinct target nucleic acid sequences of the various bacteria are labeled with a particular fluorescent DNA nanostructure having a distinct color that can be distinguished using fluorescence microscopy. Each distinct target nucleic acid molecule of a bacteria is quantified by imaging the fluorescent DNA nanostructures using the fluorescence microscopy. The individual fluorescent DNA nanostructures that are attached to the target nucleic acid molecules are identified and counted. In this manner, the relative number of single target nucleic acid molecules can be simultaneously and quickly analyzed. The relative number of individual bacterial molecules forms a quantitative fingerprint of predetermined bacterial species that is used for prognostic and therapeutic purposes.

The process of determining the quantitative fingerprint of bacterial species in a gastrointestinal microbiomic sample begins by constructing hybridized 16S rRNA polynucleotide subunits. Each 16S rRNA polynucleotide subunit is hybridized to two DNA oligonucleotide binders. First, a primary binding site is selected on the 16S subunit of a target bacterial species. The 16S subunit of the bacterial ribosome has a Svedberg sedimentation coefficient of 16S and is used to identify and quantify bacteria. The 16S rRNA genes of most bacteria in the human gastrointestinal tract have been sequenced and are listed in publicly accessible databases, such as the GenBank of the National Institute of Health. The 16S rRNA polynucleotide subunits are chosen to have more than 1000 nucleotides and less than 2000 nucleotides.

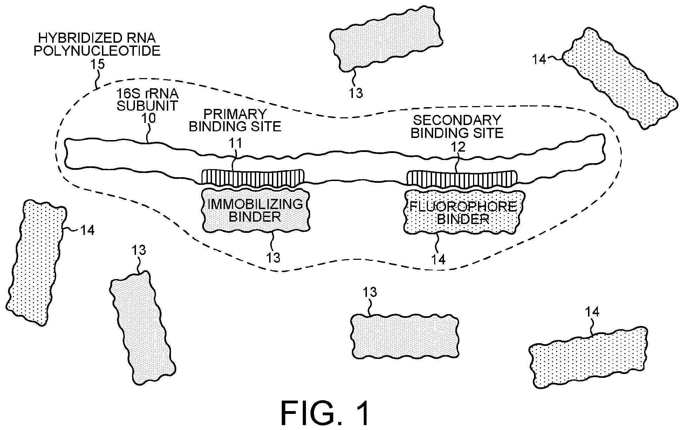

FIG. 1 illustrates an 16S rRNA subunit 10 of a target bacterial species that is to be counted using a DNA nanostructure that fluoresces a particular color. FIG. 1 schematically illustrates the locations of the nucleotide sequences that are selected as binding sites on the subunit 10. The primary binding site 11 on the rRNA subunit 10 is selected so as to include more than eighteen and less than twenty-six nucleotides and to constitute a unique sequence from among all of the 16S rRNA sequences of the various bacteria species listed in the databases. The primary binding site 11 is also selected so as to include no more than four repetitive nucleotides. In one embodiment, the primary binding site 11 on the rRNA subunit 10 is selected to be a unique sequence of twenty-three nucleotides.

FIG. 1 also illustrates a secondary binding site 12 on the rRNA subunit 10 that is selected in a manner analogous to that used to select the primary binding site 11. For example, in one embodiment, the secondary binding site 12 is also selected to be a second unique sequence of twenty-three nucleotides.

When choosing binding sites for hybridization, sequences are typically chosen that comprise 35%-55% guanine and cytosine-also described as having a 35%-55% GC content. If all nucleotides are equally present, the GC content is 50%. The GC content indicates the proportion of guanine and cytosine bases from among the four bases adenine (A), guanine (G), thymine (T) and cytosine (C) in DNA and the four bases adenine, guanine, uracil (U) and cytosine in RNA. The hydrogen bonding between GC base pairs is stronger than that between AT and AU base pairs because each GC base pair is bound by three hydrogen bonds, whereas AT and AU base pairs are bound by only two hydrogen bonds. If a binding site has a GC content of less than 35%, then the hybridization is weaker, more easily denatured, and more likely to result in a lower yield of the desired hybridized RNA polynucleotide subunits than for binding sites with higher GC content. For example, the inventors observed that hybridized RNA polynucleotides with a GC content of less than 35% are typically unbound and denatured by a washing buffer that includes a significant amount of ethanol, such as more than 5% but certainly more than 65%.

In addition to there being stronger hybridization bonding with higher GC content, there is also stronger hybridization bonding with higher complementarity between the nucleotide sequences of the binding sites on the RNA polynucleotides and the DNA oligonucleotides. Conversely, there is weaker hybridization bonding between sites exhibiting lower complementarity. In one embodiment, the washing buffer is chosen to include a very small amount of the chaotropic agent ethanol in order to denature the hybridization between nucleotide sequences that do not exhibit high complementarity, such as 95% or greater. Such a stringency-controlled washing buffer includes less than 5% ethanol and disrupts most "cross-bound" hybridizations such that the nucleotide sequences on opposite sides of the binding sites exhibit at least 95% sequence complementarity. In this embodiment, the hybridized RNA polynucleotides have a binding site with a first nucleotide sequence, and the DNA oligonucleotides have a complementary binding site with a second nucleotide sequence. After the washing step, the second nucleotide sequence of all hybridized DNA oligonucleotides exhibits at least 95% sequence complementarity with the first nucleotide sequence of the associated hybridized RNA polynucleotides. The stringency-controlled washing buffer disrupts the "cross-bound" hybridizations of the hybridized RNA polynucleotides that have less than 95% sequence complementarity.

In another step, immobilizing binders 13 are formed that include a nucleotide sequence complementary to that of the primary binding site 11 on the first rRNA subunit 10. In one embodiment, the immobilizing binders 13 are DNA oligonucleotides. In addition, fluorophore binders 14 are formed that include a nucleotide sequence complementary to that of the secondary binding site 12 on the first rRNA subunit 10. In one embodiment, the fluorophore binders 14 are also DNA oligonucleotides.

In another step, which can be performed before or after the steps disclosed above, 16S rRNA subunit polynucleotides from bacteria present in a gastrointestinal microbiomic sample from a patient are extracted. The bacteria cells in the sample are destroyed in order to purify the 16S rRNA subunits. In one example, 16S rRNA subunits from both *Coraliomargarita akajimensis* and *Bacteroides helcogenes* are extracted, as well as all of the other bacteria species present in the sample. In one implementation, the microbiomic sample is a stool sample. In other implementations, the microbiomic sample is a sewer sample, a skin swab or a saliva sample.

The steps of selecting binding sites and forming immobilizing binders and fluorophore binders are repeated in an analogous manner for the second bacterial species, in this example *Bacteroides helcogenes.* The nucleotide sequence of the second bacterial species is selected from among various bacterial species in the database. In a manner similar to that performed for the first rRNA subunit 10 of the first bacterial species, a primary binding site and a secondary binding site are selected on a second rRNA subunit of the second bacterial species. Second immobilizing binders are formed that include a nucleotide sequence complementary to that of the primary binding site on the second rRNA subunit. And second fluorophore binders are formed that include a nucleotide sequence complementary to that of the secondary binding site on the second rRNA subunit of the second bacterial species.

The extracted 16S rRNA polynucleotides 10 of the first bacterial species are then hybridized with the DNA oligonucleotides of the immobilizing binders 13 and the first fluorophore binders 14. The immobilizing binders 13, the first fluorophore binders 14, and the extracted RNA polynucleotides 10, are added to a reaction chamber, such as an annealing tube. There are sufficient immobilizing binders 13 and first fluorophore binders 14 to hybridize to all binding sites 11-12 of the RNA polynucleotides of any particular extracted bacterial species because the concentration of RNA polynucleotides for each species in the reaction chamber is orders of magnitude lower than the concentration in the chamber of immobilizing binders and fluorophore binders for the binding sites on the RNA polynucleotides of each particular species. For example, the RNA polynucleotides are added at a concentration in the pico molar (pM) range, whereas the immobilizing binders and fluorophore binders with complementary sequences to the selected binding sites of each particular bacterial species are present in the reaction chamber at a concentration in the micro molar (µM) range. In another example, the concentration of RNA polynucleotides of a particular bacterial species is 10-50 times lower than the corresponding immobilizing binders and fluorophore binders with complementary sequences.

The extracted 16S rRNA polynucleotides of the second bacterial species are also hybridized with the DNA oligonucleotides of second immobilizing binders and second fluorophore binders 14 by adding them to the reaction chamber.

In an annealing step in the reaction chamber, hybridization reactions are performed to bind the first immobilizing binders 13 to the primary binding site 11 on the first rRNA subunits 10 of the first bacterial species that are present in the microscopy chamber. Hybridization reactions are also performed to bind the fluorophore binders 14 to the secondary binding sites 12 on the first rRNA subunits 10 of the first bacterial species that are present in the reaction chamber. In addition, hybridization reactions are also performed in the reaction chamber to bind second immobilizing binders to the primary binding sites on second rRNA subunits of the second bacterial species. And hybridization reactions are performed to bind second fluorophore binders to the secondary binding sites on second rRNA subunits of the second bacterial species.

The annealing step is performed in the reaction chamber or annealing tube at 65 °C for one hour in a 4x concentration of SSC (standard sodium citrate), followed by a slow cool ramp down to room temperature. The reaction chamber can be a PCR (polymerase chain reaction) tube with a volume of 200 micro liters. The result of the annealing step is a hybridization solution that includes salt and hybridized RNA polynucleotides that are hybridized to DNA oligonucleotides. FIG. 1 shows a hybridized RNA polynucleotide 15 that is identified as the first rRNA subunit 10 of the first bacterial species. The RNA polynucleotide 10 is hybridized to an immobilizing binder 13 and to a fluorophore binder 14. In addition to hybridized RNA polynucleotides, the hybridization solution also includes DNA oligonucleotides that are not bound to any RNA polynucleotide, such as unbound immobilizing binders and fluorophore binders.

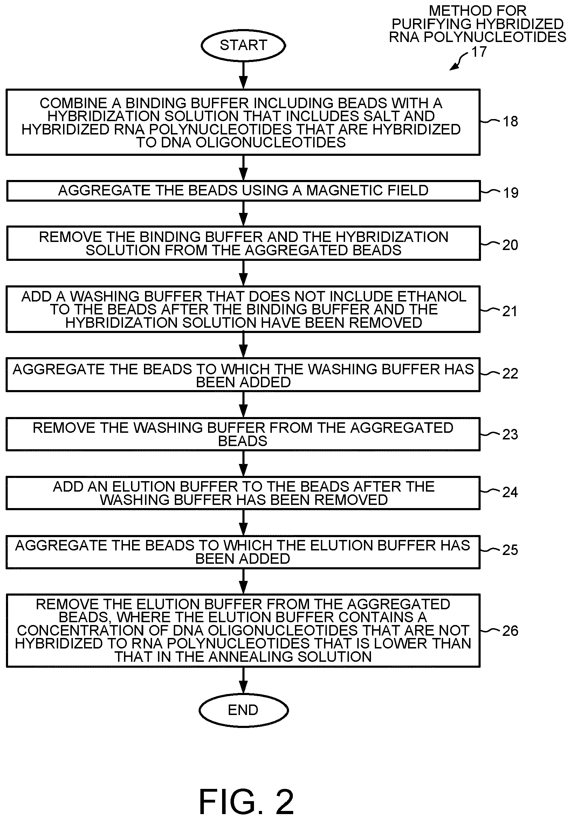

FIG. 2 is a flowchart of steps 18-26 of the method 17 for purifying hybridized RNA polynucleotides of the invention. In one application, the purified, hybridized RNA polynucleotides are used to determine a quantitative fingerprint of a subset of bacteria in a patient's gastrointestinal microbiome. The purification method of the invention uses solid-phase reverse immobilization (SPRI) to immobilize the hybridized RNA polynucleotides and separate them from unbound DNA oligonucleotides. The SPRI is implemented using paramagnetic beads, which can be made of polystyrene surrounded by a layer of magnetite that is coated with carboxyl molecules. The beads reversibly bind to the large hybridized RNA polynucleotides in the presence of a binding agent such as polyethylene glycol (PEG) and salt. Due to molecular crowding by PEG and charge shielding by the salt, the PEG and cations of the salt cause the negatively charged RNA to precipitate onto the negatively charged carboxyl groups on surface of the beads. The cations bridge the negative charges of the carboxyl groups and the negative charges of the phosphate backbone of the nucleic acids. The concentration of PEG and salt in the reaction chamber can be adjusted to select the size of the polynucleotides that are immobilized. Thus, the method 17 with its novel washing buffer is a size selective nucleic acid purification method.

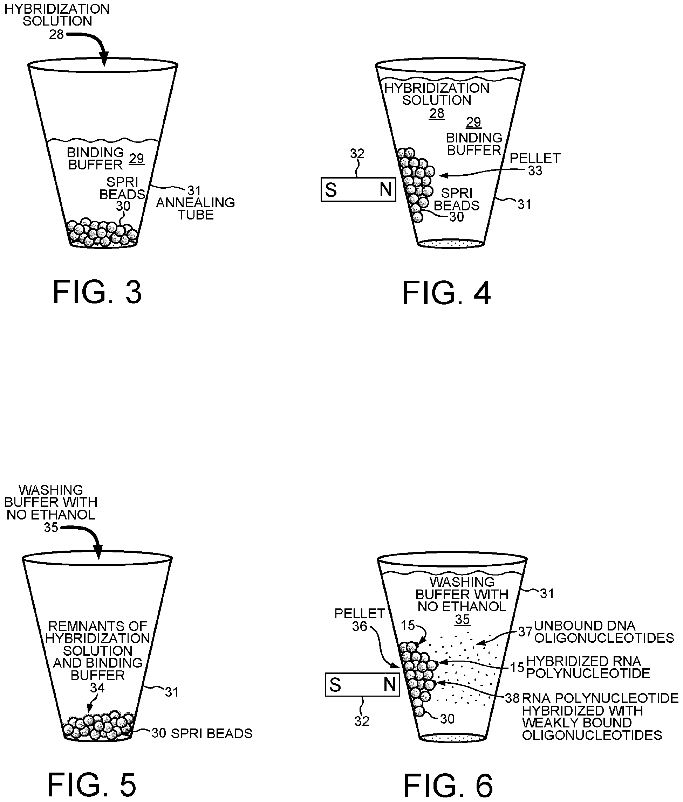

In a first step 18, the hybridization solution 28 resulting from the annealing step is combined with a binding buffer 29 that includes SPRI paramagnetic beads 30. FIG. 3 illustrates the binding buffer 29 including beads 30 being combined with the hybridization solution 28 in an annealing tube 31, such as a PCR tube. One volume of the hybridization solution 28 is combined with one volume of the binding buffer 29. The hybridization solution includes salt and the hybridized RNA polynucleotides 15 that are hybridized to DNA oligonucleotides, in this case the immobilizing binders 13 and the fluorophore binders 14. The binding buffer includes beads at 0.1% weight per volume (w/v). The binding buffer can be made using nuclease free water. The binding buffer also includes 18% w/v polyethylene glycol (PEG 8000), 1 M sodium chloride (NaCl), 10 mM Tris-HCl, 0.1 mM EDTA pH 8, and 0.054% v/v Tween 20. Tris stands for 3-hydroxypropyltriazolylmethylamine, EDTA stands for enthylenediaminetetraacetic acid, and Tween 20 stands for polysorbate 20 (polyoxyethylene (20) sorbitan monolaurate). The pH of the buffer with Tris is adjusted by the HCl. EDTA chelates divalent cations such as magnesium, which can contribute to polynucleotide degradation. Tween 20 is a non-ionic detergent that reduces the adsorption of polynucleotides to plastics, such as the plastic used to make pipettes. The concentration of PEG is chosen so as to bind RNA polynucleotides having 1000 or more nucleotides (base pairs) to the beads. Generally, the higher the concentration of PEG, the smaller the size of polynucleotide fragments that become bound to the surface of the carboxylated magnetic beads.

After the binding buffer 29 including beads is combined with the hybridization solution 28, the combined bead suspension is carefully pipetted up and down to mix the beads with the hybridization solution and binding buffer. Alternatively, the PCR tube 31 containing the combined bead suspension can be inverted several times. The total volume of the combined bead suspension should not exceed 200 µl. The combined bead suspension is then incubated for 10 minutes at room temperature.

In step 19, the beads 30 are aggregated using a magnetic field. FIG. 4 illustrates the PCR tube 31 containing the combined bead suspension being placed on a magnetic rack, where the beads are aggregated at the magnet 32 into a pellet 33 for five minutes at room temperature.

In step 20, the binding buffer and the hybridization solution are removed from the aggregated beads. Without disturbing the bead pellet, the supernatant containing unbound oligonucleotides is removed and discarded. This is performed by pipetting out the supernatant slowly so as not to dislodge any beads from the magnet.

Although most of the unbound immobilizing binders 13 and fluorophore binders 14 have been separated from the bead pellet with the removal of the binding buffer and the hybridization solution, some unhybridized immobilizing binders and fluorophore binders are still present in the small amount of liquid 34 that surrounds the beads. The next task is to wash the beads 30 of the DNA oligonucleotides that are not hybridized to RNA polynucleotides that are immobilized onto the beads due to their large size. In the washing task, the PCR tube 31 is removed from the magnetic rack and the beads are resuspended in a washing buffer 35.

In step 21, a washing buffer 35 is added to the beads 30 after the binding buffer 29 and the hybridization solution 28 have been removed. FIG. 5 illustrates the washing buffer 35 being added to the beads 30 and the remnants 34 of hybridization solution 28 and binding buffer 29 on the surface of the beads. The washing buffer 35 includes no ethanol, which would separate the hybridized immobilizing binders 13 and fluorophore binders 14 from the binding sites 11-12 on the hybridized RNA polynucleotides 10. The PCR tube 31 is taken off the magnetic separator, and 100 µl of washing buffer and 100 µl of water are added to the PCR tube to resuspend the beads 30. The beads 30 and washing buffer 35 are pipetted up and down several times, or the PCR tube can be inverted several times. The washing buffer includes PEG 8000, NaCl, EDTA, a Tris HCl buffering agent, and a Tween 20 surfactant. The 2x concentration washing buffer before dilution with water includes 18% w/v PEG 8000, 1 M NaCl, 10 mM Tris-HCl, 0.1 mM EDTA pH 8, and 0.054% v/v Tween 20. Thus, the 1x concentration washing buffer includes PEG 8000 at a weight per volume between 8% and 10%. After 100 µl of washing buffer and 100 µl of water are added to the PCR tube, the washing buffer 35 includes 9% w/v PEG 8000, 0.5 M NaCl, 5 mM Tris-HCl, 0.05 mM EDTA pH 8, and 0.027% v/v Tween 20. The washing buffer 35 includes no ethanol, as is commonly used in washing buffers for SPRI bead purification.

Washing buffers used in conventional SPRI bead purification typically include 65-85% ethanol, which immobilizes the larger polynucleotides and allows impurities such as salts, proteins and smaller oligonucleotides to be washed off. Using concentrations of ethanol in the washing buffer less than 65% reduces the yield of the purified polynucleotides because their solubility increases with lower ethanol concentrations. Using concentrations of ethanol in the washing buffer higher than 85% reduces the purity because the impurities are less soluble in higher ethanol concentrations. Conventional wash buffers use 65-85% ethanol in order to achieve "ethanol precipitation" in which the polynucleotides remain immobilized on the SPRI beads because ethanol has a lower polarity than water and screens charges less, allowing cations to form ionic bonds with the phosphate groups of the polynucleotides and precipitate them out of solution. However, the inventors recognized that using a washing buffer as part of SPRI bead purification with high ethanol concentrations to purify hybridized polynucleotides would unbind and denature the hybridization and lower the yield of hybridized RNA polynucleotides that precipitate onto the beads. The solution is to purify the hybridized RNA polynucleotides without using ethanol or by using only a low concentration of ethanol, such as 5% or less. In addition to having no, or only a low concentration, of ethanol, the washing buffer has a high salt concentration and a crowding agent, such as PEG, to keep the hybridized RNA polynucleotides on the beads. The DNA oligonucleotides (immobilizing binders 13 and fluorophore binders 14) that are not hybridized to RNA polynucleotides are removed from the sample of hybridized RNA polynucleotides 15 together with the washing buffer that includes only a low concentration, if any, of ethanol. Thus, the method of purifying hybridized RNA polynucleotides in a sample according to the invention includes combining the sample and a washing buffer that includes (1) beads to which the hybridized RNA polynucleotides are bound and (2) DNA oligonucleotides that are not hybridized to RNA polynucleotides. The washing buffer includes ethanol at a concentration of 5% or less.

After the washing buffer 35 is added to the beads 30 in step 21, the liquid is collected in the PCR tube 31 by pulsing briefly in a centrifuge, for example, spinning for two seconds. The PCR tube 31 containing the beads 30 and washing buffer 35 is allowed to stand for two minutes at room temperature.

In step 22, the beads to which the washing buffer 35 has been added are aggregated using a magnet 32. The tube 31 is placed back on the magnetic rack, where the beads are aggregated at the magnet 32 into a pellet 36 for two minutes at room temperature. FIG. 6 illustrates the PCR tube 31 containing the beads 30 and washing buffer 35 being placed on the magnetic rack, where the beads are aggregated into the pellet 36. Unbound DNA oligonucleotides 37 (immobilizing binders 13 and fluorophore binders 14) that are not hybridized to RNA polynucleotides 10 are soluble and suspended in the washing buffer. On the other hand, hybridized RNA polynucleotides 15 have precipitated onto the beads 30.

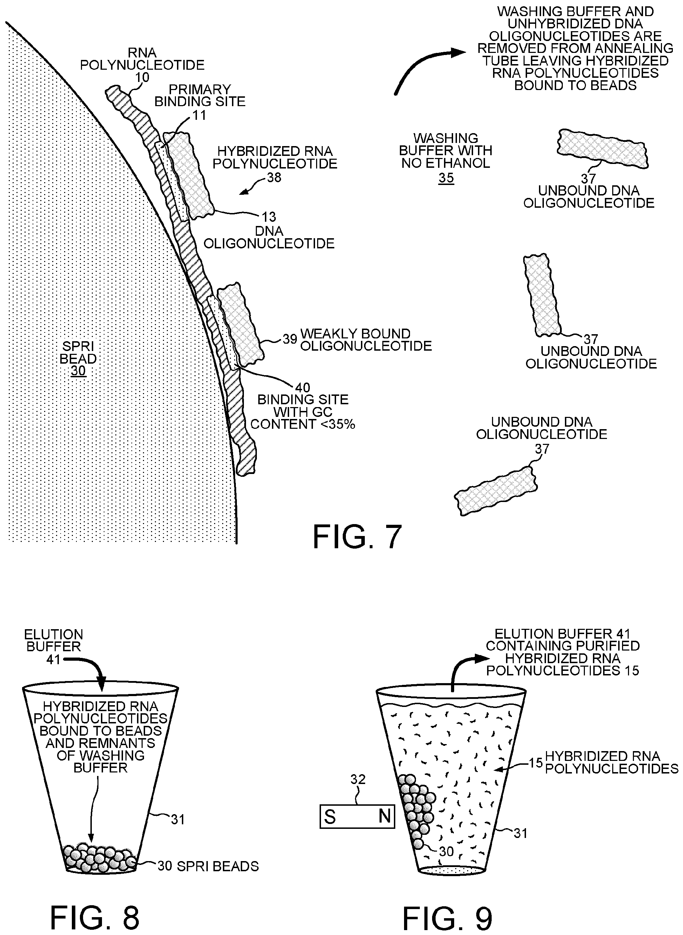

FIG. 7 illustrates the situation in which some of the hybridized RNA polynucleotides 38 are hybridized to weaker bound oligonucleotides 39. For example, RNA polynucleotides 38 that are hybridized to DNA oligonucleotides at binding sites 40 having a GC content of less than 35% typically exhibit weaker hybridization. The inventors observed that such hybridized DNA oligonucleotides 38 with weakly bound DNA oligonucleotides 39 were largely unbound and denatured by washing buffers that included more than 65% ethanol. This resulted in lower yields of hybridized RNA polynucleotide subunits, especially those hybridized polynucleotides selected to have at least one binding site with a GC content of less than 35%. If a binding site has a GC content of less than 35%, then the hybridization is weaker, more easily denatured, and more likely to result in a lower yield of the desired hybridized RNA polynucleotide subunits than for binding sites with higher GC content. However, the washing buffer 35 that includes no ethanol did not largely unbind RNA polynucleotides 38 hybridized to DNA oligonucleotides even at binding sites 40 having a GC content of less than 35%. Thus, the purification method 17 of the invention can be used to separate hybridized RNA polynucleotides 38 from unbound DNA oligonucleotides 37 even when one or more of the binding sites 40 have been selected to have a GC content of less than 35%. This allows more flexibility in choosing binding sites for the hybridization of the 16S rRNA polynucleotides.

Using the washing buffer 35 that includes no ethanol also allows acceptably large yields of purified hybridized RNA polynucleotide subunits to be achieved for hybridized polynucleotides with binding sites all having a GC content of 35% or more.

In step 23, the washing buffer 35 is removed from the aggregated beads. After the beads 30 are aggregated into a pellet 36 in step 22, the washing buffer 35 together with the unbound DNA oligonucleotides 37 are removed from the aggregated beads of pellet 36. FIG. 7 illustrates the washing buffer 35 and the unhybridized DNA oligonucleotides being removed from the annealing tube 31 leaving the hybridized RNA polynucleotides bound to the beads. Without disturbing the bead pellet 36, the supernatant containing unbound oligonucleotides 37 is removed and discarded. This is performed by pipetting out the supernatant carefully so as not to dislodge any beads from the magnet 32.

After the washing buffer 35 is removed, the washing steps 21-23 are repeated at least once.

The next task is to remove the purified hybridized RNA polynucleotides 38 from the SPRI beads 30.

In step 24, an elution buffer 41 is added to the beads 30, as illustrated in FIG. 8, after the washing buffer 35 has been removed. After the PCR tube 31 is removed from the magnetic rack, hybridized RNA polynucleotides 15 bound to beads 30 and remnants of the second wash of washing buffer 35 are present at the bottom of the PCR tube 31. The beads 30 are resuspended by adding 30 µl of a 1x concentration of elution buffer 41. The elution buffer 41 includes a 4x concentration of SSC (standard sodium citrate) at pH 7.0, a 5x concentration of Denhardt's Solution, 5% w/v of dextran sulfate, and 0.1% v/v of Tween 20. The bead suspension is carefully pipetted up and down to mix the beads with the elution buffer 41. The PCR tube 31 is briefly pulsed in a tabletop centrifuge by spinning for two seconds. The elution buffer 41 and beads are allowed to incubate for 15 minutes at room temperature.

In step 25, the beads to which the elution buffer has been added are aggregated by placing the PCR tube 31 back onto the magnetic rack. The beads are allowed to aggregate at the magnet 32 for two minutes at room temperature.

In step 26, the elution buffer 41, which is the supernatant, is removed from the aggregated beads, as illustrated in FIG. 9. The elution buffer 41 that is removed contains the purified hybridized RNA polynucleotides 15. The elution buffer 41 that is removed also contains a concentration of DNA oligonucleotides that are not hybridized to RNA polynucleotides that is lower than that present in the hybridization solution 28. After removal, the supernatant can be either stored briefly at 4°C or added directly to the next reaction chamber for hybridization to the fluorescent DNA nanostructures 42. The hybridized RNA polynucleotides 15 have now been purified according to method 17 of the invention and without unbinding the DNA oligonucleotides from the RNA polynucleotides and without reducing the yield of the purified hybridized RNA polynucleotides 15.

In subsequent tasks, the fluorescing DNA nanostructures 42 corresponding to each bacterial species are attached to the complementary DNA oligonucleotide fluorophore binders 14 that are hybridized to the 16S rRNA subunits 10 for the corresponding bacterial species. In addition, the DNA oligonucleotide immobilizing binders 13 that are hybridized to the 16S rRNA subunits 10 are attached to immobilizing oligonucleotides 43 that are in turn indirectly attached to the surface of the slide or microscopy chamber and thereby immobilize the hybridized RNA polynucleotides 15 and attached fluorescing DNA nanostructure 42.

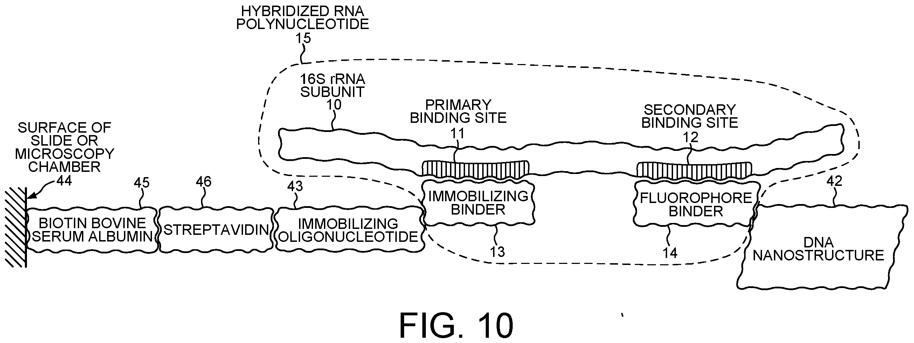

FIG. 10 shows a hybridized RNA polynucleotide 15 that is attached to a fluorescing DNA nanostructure 42 as well as to an immobilizing oligonucleotide 43. In one implementation, the immobilizing oligonucleotide 43 is attached to the glass surface 44 of the slide or microscopy chamber through protein-ligand (biotin-streptavidin) coupling. Biotin bovine serum albumin (bBSA) 45 supplies the biotin that passivates the surface 44 and attaches to streptavidin 46. The surface 44 of the slide or microscopy chamber is thereby coated with streptavidin 46, which is then hybridized to the immobilizing oligonucleotide 43. The immobilizing oligonucleotides 43 also include biotin, which binds to the streptavidin 46. In a second implementation, the immobilizing oligonucleotides 43 are attached to the surface 44 of the slide or microscopy chamber by being attached directly to the biotin bovine serum albumin (bBSA) 45, and there is no intermediary streptavidin.

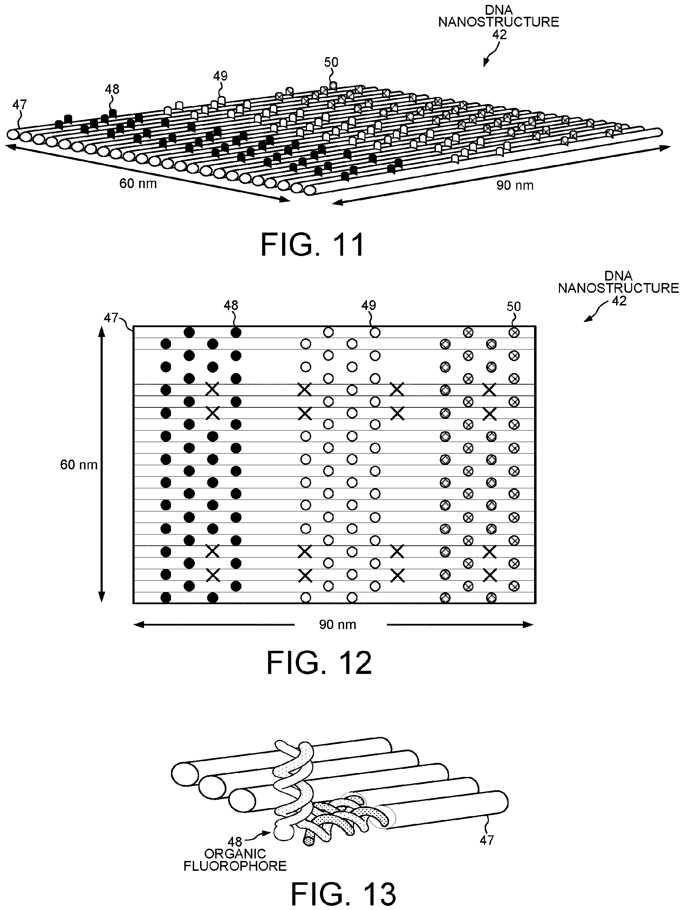

One task in a method of generating a quantitative fingerprint of bacterial abundance in a patient's gastrointestinal tract is to attach the first fluorophore binders 14 to first DNA nanostructures 42 that correspond to a first bacteria strain, such as *Coraliomargarita akajimensis* as mentioned in the example above. The DNA nanostructures each include parallel DNA double helices that form a flat shape. FIG. 11 is a perspective schematic diagram of an exemplary DNA nanostructure of the type used in the method for quantifying bacteria. For additional details on the method for quantifying bacteria, see U.S. Patent Application serial number 17/576,865 entitled "Method of Determining a Quantitative Fingerprint of a Subset of Bacteria in a Person's Gastrointestinal Microbiome," published as U.S. Patent Publication No. 2023/0227892, which is incorporated herein by reference in its entirety.

The DNA nanostructures used in the bacteria quantifying method are made by folding double-helix DNA strands into a flat structure, which is only slightly curved and has a rippled surface. The DNA nanostructures have maximum lengths of less than 110 nm. First DNA nanostructure 42, which is used to quantify the first bacterial strain, is rectangular with dimensions of 60nm by 90nm. Longer DNA rail strands are held in place by shorter rung strands that connect locations on the longer folded rail strands. FIG. 11 shows that first DNA nanostructure 42 is formed from twenty-four, parallel DNA rail strands, such as rail strand 47 that forms the back side of the rectangle. The DNA rail strands are held in the rectangular shape by 184 rung strands. FIG. 12 is a top view of first DNA nanostructure 42.

Many fluorescence dye molecules (called organic fluorophores) are attached to each DNA nanostructure. The organic fluorophores are attached to the DNA rail strands using connectors formed by twenty-one nucleotides that attach to the 3'-end of the DNA strand. A complementary connector attaches to the DNA nanostructure. FIGS. 11-12 illustrate where the first DNA nanostructure 42 is attached to a first organic fluorophore 48 with a first color, a second organic fluorophore 49 with a second color, and a third organic fluorophore 50 with a third color. Each of the exemplary organic fluorophores 48-50 is attached to the DNA rail strand 47. FIG. 13 shows more detail of how first organic fluorophore 48 is attached to the double-helix DNA strand of rail strand 47.

In one implementation, the first color is red, and first organic fluorophore 48 is Atto 647N. The second color is green, and second organic fluorophore 49 is Cy3. The third color is blue, and third organic fluorophore 50 is Atto 488. In order for the fluorescence microscopy system better to distinguish the wavelengths from the various fluorescence colors, fluorophores are chosen that fluoresce at wavelengths that differ by at least 25nm. For example, the three organic fluorophores on DNA nanostructure 42 are chosen such that the second organic fluorophore 49 has a wavelength of maximum absorption that is at least 25nm larger than the wavelength of maximum emission of the third organic fluorophore 50, and the first organic fluorophore 48 has a wavelength of maximum absorption that is at least 25nm larger than the wavelength of maximum emission of the second organic fluorophore 49. In the implementation shown in FIGS. 11-12, there are forty-four fluorophore molecules of each color disposed within the rectangular DNA nanostructure 42 of 60nm x 90nm. Because the size of the DNA nanostructure is so small, the variously colored light emitted by different fluorophores combines and is perceived by a fluorescence microscopy system as a single multi-colored spot. Thus, the first DNA nanostructures exhibit a first fluorophore color produced by a first combination of intensities of the first color, the second color and the third color.

The relative intensities of the differently colored light contribute to the single combined color emitted from each DNA nanostructure. The intensity level of the light of each color can be adjusted by attaching a discrete number of that color fluorophore to the each DNA nanostructure. The number of possibilities for the single combined color emitted from each DNA nanostructure is N=L^C, where L is the number of intensity levels of each fluorophore color, and C is the number of fluorophore colors. For example, with two fluorophore colors and three intensity levels of those colors, nine single combined colors are possible. With three fluorophore colors and two intensity levels of those colors, eight single combined colors are possible. With three fluorophore colors and four intensity levels of those colors, sixty-four single combined colors are possible. And with three fluorophore colors and five intensity levels of those colors, 125 single combined colors are possible (actually 124 colors because zero intensity for all three colors is not a color). In the example shown in FIGS. 11-12, the three differently colored fluorophores have the same number of fluorophore molecules and, therefore, emit the same intensity of each of the three colors. The resulting combined color is just one of the 124 possible combined colors that can be generated with three original colors and four intensities plus black (zero intensity). Using this intensity and color implementation, a panel of 124 bacteria can be quantified using the small DNA nanostructures all having the same basic configuration.

In the implementation shown in FIGS. 11-12, there are forty-eight positions for fluorophore molecules in the matrix forming the stripe for each color. However, four of the forty-eight positions are not occupied by fluorophore molecules because those positions are used to attach binding oligonucleotides that connect the DNA nanostructure 42 to the associated fluorophore binder 14. Two matrix positions are used to attach each binding oligonucleotide. The fluorophore binders 14 include a nucleotide sequence complementary to that of the binding oligonucleotides that protrude from the surface of the DNA nanostructure 42. In FIG. 12, there are eight pairs of attachment positions marked by Xs at which the first fluorophore binders 14 are attached to the first DNA nanostructures 42.

Returning to the beginning of the task of the bacteria quantifying method involving attaching fluorophore binders to DNA nanostructures, second fluorophore binders are attached to second DNA nanostructures that correspond to a second bacteria strain, such as *Bacteroides helcogenes* as mentioned in the example above. The steps that were performed for the first bacterial species are repeated in an analogous manner for the second bacterial species. The steps that are repeated for the second bacterial species are performed simultaneously with the steps for the first bacterial species as opposed to sequentially.

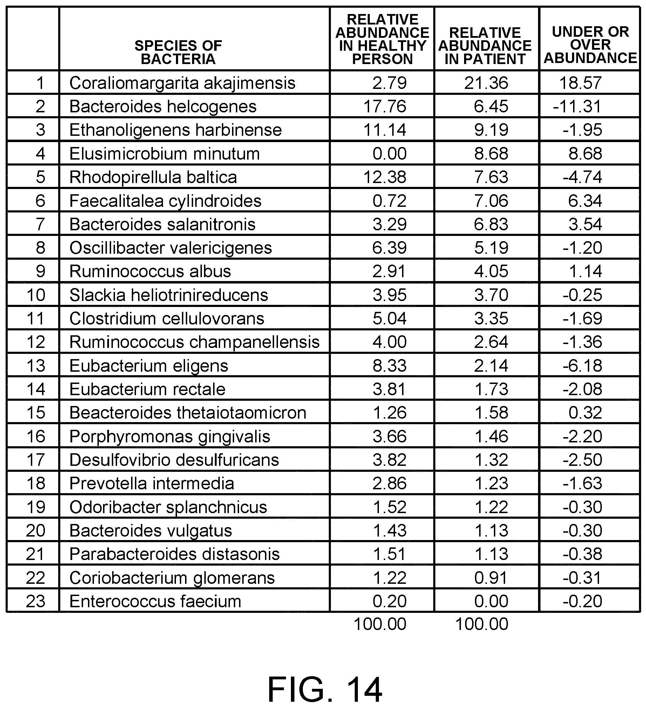

The second bacterial species of the subset is selected from various bacterial species in the database. For example, FIG. 14 lists twenty-three bacterial species of a predetermined subset of species that are to be quantified by the bacteria quantifying method. Purification of the hybridized bacterial rRNA polynucleotides without losing any significant yield due to denaturing caused by washing with ethanol is essential to generating an accurate quantitative fingerprint of the relative abundance of each of the twenty-three species of bacteria in a patient's gastrointestinal tract. FIG. 14 also lists the relative abundance of each of the bacterial species determined by the bacteria quantifying method to be present in the patient's gastrointestinal tract. In performing the steps of the method for the second bacterial species, a primary binding site is selected on a second rRNA subunit of the second bacterial species. The primary binding site is for hybridizing an immobilizing binder DNA oligonucleotide. A secondary binding site is selected on the second rRNA subunit of the second bacterial species. The secondary binding site is for hybridizing a fluorophore binder DNA oligonucleotide. The second immobilizing binders are formed that include a nucleotide sequence complementary to that of the primary binding site on the second rRNA subunit. Second fluorophore binders are formed that include a nucleotide sequence complementary to that of the secondary binding site on the second rRNA subunit.

The annealing step is performed for 16S rRNA subunits of the second bacterial species with a hybridization reaction that binds the second immobilizing binders to the primary binding site on the second rRNA subunits of the second bacterial species that are present in the reaction chamber. Hybridization reactions are also performed to bind fluorophore binders to secondary binding sites on the second rRNA subunits of the second bacterial species that are present in the reaction chamber. The annealing step is performed for the second bacterial species analogously to the manner in which the annealing step was performed for the first bacterial species. The result of the annealing step is a hybridization solution that includes salt and hybridized RNA polynucleotides that are hybridized to DNA oligonucleotides.

Steps 18-26 of the I method 17 for purifying hybridized RNA polynucleotides of the invention are then performed on the hybridization solution resulting from the annealing step that hybridizes immobilizing binders and fluorophore binders to the second rRNA subunits of the second bacterial species. Then the remainder of the steps of the bacteria quantifying method is performed using the purified, hybridized RNA polynucleotides of the second bacterial species.

Finally, the bacteria quantifying method including the method 17 for purifying hybridized RNA polynucleotides of the invention is performed on the remainder of the twenty-three bacterial species in the predetermined subset of species. Many steps of the methods can be performed for all of the bacterial species simultaneously in the same reaction chambers and PCR tubes.

After the hybridized RNA polynucleotides attached to the fluorescing DNA nanostructures corresponding to each bacterial species have been immobilized on the surface of the slide or microscopy chamber, image analysis is performed to detect and count the number of DNA nanostructures that fluoresce the particular fluorophore color associated with each bacterial species. Thus, each of the spots with a particular fluorophore color corresponding to a particular bacterial species is counted in order to generate the quantitative fingerprint of bacterial species in the gastrointestinal microbiomic sample from the patient. The results of the bacteria quantifying method are shown in FIG. 14.

Below is a list of method embodiments. Those will be indicated by with the letter "M". Whenever such embodiments are referred to, this will be done by referring to "M" embodiments.
M1. A method of purifying hybridized RNA polynucleotids, comprising:
   combining a binding buffer including beads with a hybridization solution, wherein the hybridization solution includes salt and the hybridized RNA polynucleotides that are hybridized to DNA oligonucleotides;
   aggregating the beads using a magnetic field;
   removing the binding buffer and the hybridization solution from the aggregated beads;
   adding a washing buffer to the beads after the binding buffer and the hybridization solution have been removed, wherein the washing buffer includes no ethanol;
   aggregating the beads to which the washing buffer has been added;
   removing the washing buffer from the aggregated beads;
   adding an elution buffer to the beads after the washing buffer has been removed;
   aggregating the beads to which the elution buffer has been added; and
   removing the elution buffer from the aggregated beads, wherein the elution buffer contains a concentration of DNA oligonucleotides that are not hybridized to RNA polynucleotides that is lower than that in the hybridization solution.
M2. The method of the preceding embodiment, wherein each of the hybridized RNA polynucleotides has a binding site that includes more than 18 nucleotides and less than 26 nucleotides to which a DNA oligonucleotide is hybridized.
M3. The method of the preceding embodiment, wherein the binding site includes nucleotides comprising less than 35% Guanine and Cytosine.
M4. The method of any of embodiments 2 to 3, wherein the binding site has a first nucleotide sequence, wherein each of the DNA oligonucleotides has a complementary binding site having a second nucleotide sequence, and wherein the second nucleotide sequence exhibits at least 95% sequence complementarity with the first nucleotide sequence.
M5. The method of any of embodiments 1 to 4, wherein the hybridized RNA polynucleotides are 16S ribosomal RNA polynucleotides.
M6. The method of any of embodiments 1 to 5, wherein the beads are solid-phase reversible immobilization (SPRI) paramagnetic beads.
M7. The method of any of embodiments 1 to 6, wherein the beads are present in the binding buffer at a weight per volume of approximately 0.1%.
M8. The method of any of embodiments 1 to 7, wherein the washing buffer includes PEG 8000, NaCl, EDTA, a Tris-HCl buffering agent, and a Tween 20 surfactant.
M9. The method of embodiment 8, wherein the washing buffer includes PEG 8000 at a weight per volume between 8% and 10%.
M10. The method of claim any of embodiments 1 to 9, wherein each of the hybridized RNA polynucleotides is comprised of 1000 or more nucleotides but 2000 or fewer nucleotides.
M11. A method of purifying hybridized RNA polynucleotides, comprising:
   adding a hybridization solution to a binding buffer and beads, wherein the hybridization solution includes the hybridized RNA polynucleotides that are hybridized to DNA oligonucleotides;
   aggregating the beads using a magnetic field;
   removing the binding buffer and the hybridization solution from the aggregated beads;
   adding a washing buffer to the beads after the binding buffer and the hybridization solution have been removed, wherein the washing buffer includes less than 5% ethanol;
   aggregating the beads to which the washing buffer has been added;
   removing the washing buffer from the aggregated beads;
   adding an elution buffer to the beads after the washing buffer has been removed;
   aggregating the beads to which the elution buffer has been added; and
   removing the elution buffer from the aggregated beads, wherein the elution buffer contains purified hybridized RNA polynucleotides from which substantially all DNA oligonucleotides that are not hybridized to RNA polynucleotides have been removed.
M12. The method of embodiment 11, wherein each of the hybridized RNA polynucleotides has a binding site that includes more than 18 nucleotides and less than 26 nucleotides to which a DNA oligonucleotide is hybridized.
M13. The method of embodiment 12, wherein the binding site includes nucleotides comprising less than 35% Guanine and Cytosine.
M14. The method of embodiment 12 and/or 13, wherein the binding site has a first nucleotide sequence, wherein each of the DNA oligonucleotides has a complementary binding site having a second nucleotide sequence, and wherein the second nucleotide sequence exhibits at least 95% sequence complementarity with the first nucleotide sequence.
M15. A method of purifying hybridized RNA polynucleotides in a sample, comprising:
   adding a washing buffer to the sample, wherein the sample comprises beads to which the hybridized RNA polynucleotides are bound and DNA oligonucleotides that are not hybridized to RNA polynucleotides, and wherein the washing buffer comprises ethanol at a concentration of 5% or less; and
   removing from the sample at least some of the washing buffer and at least some of the DNA oligonucleotides that are not hybridized to RNA polynucleotides.
M16. The method of embodiment 15, wherein the beads are solid-phase reversible immobilization (SPRI) paramagnetic beads.
M17. The method of embodiment 15 and/or 16, wherein each of the hybridized RNA polynucleotides has a binding site that includes more than 18 nucleotides and less than 26 nucleotides to which a DNA oligonucleotide is hybridized.
M18. The method of embodiment 17, wherein the binding site includes nucleotides comprising less than 35% Guanine and Cytosine.
M19. The method of embodiment 17 and/or 18, wherein the binding site has a first nucleotide sequence, wherein each of the DNA oligonucleotides has a complementary binding site having a second nucleotide sequence, and wherein the second nucleotide sequence exhibits at least 95% sequence complementarity with the first nucleotide sequence.
M20. The method of any of embodiment 15 to 19, wherein the washing buffer includes PEG 8000 at a weight per volume between 8% and 10%.
M21. A method of purifying hybridized RNA polynucleotides, comprising:
   adding a hybridization solution to a binding buffer and beads, wherein the hybridization solution includes the hybridized RNA polynucleotides that are hybridized to DNA oligonucleotides, and wherein a first binding site with a first nucleotide sequence on the RNA polynucleotides is hybridized to a second binding site with a second nucleotide sequence on the DNA oligonucleotides;
   aggregating the beads using a magnetic field;
   removing the binding buffer and the hybridization solution from the aggregated beads;
   adding a washing buffer to the beads after the binding buffer and the hybridization solution have been removed, wherein the washing buffer has a temperature and a stringency, and wherein the stringency of the washing buffer is adjusted to provide a melting temperature between the first binding site with the first nucleotide sequence and the second binding site with the second nucleotide sequence of 5 to 10 degrees Celsius above the temperature of the washing buffer;
   aggregating the beads to which the washing buffer has been added;
   removing the washing buffer from the aggregated beads;
   adding an elution buffer to the beads after the washing buffer has been removed;
   aggregating the beads to which the elution buffer has been added; and
   removing the elution buffer from the aggregated beads, wherein the elution buffer contains purified hybridized RNA polynucleotides from which substantially all DNA oligonucleotides that are not hybridized to RNA polynucleotides have been removed.

Below is a list of use embodiments. Those will be indicated with a letter "U". Whenever such embodiments are referred to, this will be done by referring to "U" embodiments.
U1. Use of the method of the invention in a method for determining a quantitative fingerprint of a predetermined subset of bacterial species in a gastrointestinal tract sample of a patient.
U2. The use of the preceding embodiment, wherein small DNA nanostructures are used to detect and count single nucleic acid molecules of the various target bacterial species.
U3. The use of the preceding embodiments, wherein the DNA nanostructure is attached that fluoresces a predetermined fluorophore color to a ribosomal RNA (rRNA) polynucleotide subunit of the target bacterial species that corresponds to the predetermined color.

Although the present invention has been described in connection with certain specific embodiments for instructional purposes, the present invention is not limited thereto. Accordingly, various modifications, adaptations, and combinations of various features of the described embodiments can be practiced without departing from the scope of the invention as set forth in the claims.

## Claims

1. A method of purifying hybridized RNA polynucleotides, comprising:
combining a binding buffer including beads with a hybridization solution, wherein the hybridization solution includes salt and the hybridized RNA polynucleotides that are hybridized to DNA oligonucleotides;
aggregating the beads using a magnetic field;
removing the binding buffer and the hybridization solution from the aggregated beads;
adding a washing buffer to the beads after the binding buffer and the hybridization solution have been removed, wherein the washing buffer includes no ethanol;
aggregating the beads to which the washing buffer has been added;
removing the washing buffer from the aggregated beads;
adding an elution buffer to the beads after the washing buffer has been removed;
aggregating the beads to which the elution buffer has been added; and
removing the elution buffer from the aggregated beads, wherein the elution buffer contains a concentration of DNA oligonucleotides that are not hybridized to RNA polynucleotides that is lower than that in the hybridization solution.

2. The method of claim 1, wherein each of the hybridized RNA polynucleotides has a binding site that includes more than 18 nucleotides and less than 26 nucleotides to which a DNA oligonucleotide is hybridized.

3. The method of claim 2, wherein the binding site includes nucleotides comprising less than 35% Guanine and Cytosine.

4. The method of any of claims 2 to 3, wherein the binding site has a first nucleotide sequence, wherein each of the DNA oligonucleotides has a complementary binding site having a second nucleotide sequence, and wherein the second nucleotide sequence exhibits at least 95% sequence complementarity with the first nucleotide sequence.

5. The method of any of claims 1 to 4, wherein the hybridized RNA polynucleotides are 16S ribosomal RNA polynucleotides.

6. The method of any of claims 1 to 5, wherein the beads are solid-phase reversible immobilization (SPRI) paramagnetic beads.

7. The method of any of claims 1 to 6, wherein the beads are present in the binding buffer at a weight per volume of approximately 0.1%.

8. The method of any of claims 1 to 7, wherein the washing buffer includes PEG 8000, NaCl, EDTA, a Tris-HCl buffering agent, and a Tween 20 surfactant.

9. The method of claim 8, wherein the washing buffer includes PEG 8000 at a weight per volume between 8% and 10%.

10. The method of any of claims 1 to 9, wherein each of the hybridized RNA polynucleotides is comprised of 1000 or more nucleotides but 2000 or fewer nucleotides.

11. A method of purifying hybridized RNA polynucleotides, comprising:
adding a hybridization solution to a binding buffer and beads, wherein the hybridization solution includes the hybridized RNA polynucleotides that are hybridized to DNA oligonucleotides;
aggregating the beads using a magnetic field;
removing the binding buffer and the hybridization solution from the aggregated beads;
adding a washing buffer to the beads after the binding buffer and the hybridization solution have been removed, wherein the washing buffer includes less than 5% ethanol;
aggregating the beads to which the washing buffer has been added;
removing the washing buffer from the aggregated beads;
adding an elution buffer to the beads after the washing buffer has been removed;
aggregating the beads to which the elution buffer has been added; and
removing the elution buffer from the aggregated beads, wherein the elution buffer contains purified hybridized RNA polynucleotides from which substantially all DNA oligonucleotides that are not hybridized to RNA polynucleotides have been removed.

12. The method of claim 11, wherein each of the hybridized RNA polynucleotides has a binding site that includes more than 18 nucleotides and less than 26 nucleotides to which a DNA oligonucleotide is hybridized.

13. The method of claim 12, wherein the binding site includes nucleotides comprising less than 35% Guanine and Cytosine.

14. The method of claim(s) 12 and/or 13, wherein the binding site has a first nucleotide sequence, wherein each of the DNA oligonucleotides has a complementary binding site having a second nucleotide sequence, and wherein the second nucleotide sequence exhibits at least 95% sequence complementarity with the first nucleotide sequence.

15. A method of purifying hybridized RNA polynucleotides in a sample, comprising:
adding a washing buffer to the sample, wherein the sample comprises beads to which the hybridized RNA polynucleotides are bound and DNA oligonucleotides that are not hybridized to RNA polynucleotides, and wherein the washing buffer comprises ethanol at a concentration of 5% or less; and
removing from the sample at least some of the washing buffer and at least some of the DNA oligonucleotides that are not hybridized to RNA polynucleotides.

16. The method of claim 15, wherein the beads are solid-phase reversible immobilization (SPRI) paramagnetic beads.

17. The method of claim(s) 15 and/or 16, wherein each of the hybridized RNA polynucleotides has a binding site that includes more than 18 nucleotides and less than 26 nucleotides to which a DNA oligonucleotide is hybridized.

18. The method of claim 17, wherein the binding site includes nucleotides comprising less than 35% Guanine and Cytosine.

19. The method of claim(s) 17 and/or 18, wherein the binding site has a first nucleotide sequence, wherein each of the DNA oligonucleotides has a complementary binding site having a second nucleotide sequence, and wherein the second nucleotide sequence exhibits at least 95% sequence complementarity with the first nucleotide sequence.

20. The method of any of claims 15 to 19, wherein the washing buffer includes PEG 8000 at a weight per volume between 8% and 10%.

21. A method of purifying hybridized RNA polynucleotides, comprising:
adding a hybridization solution to a binding buffer and beads, wherein the hybridization solution includes the hybridized RNA polynucleotides that are hybridized to DNA oligonucleotides, and wherein a first bonding site with a first nucleotide sequence on the RNA polynucleotides is hybridized to a second binding site with a second nucleotide sequence on the DNA oligonucleotides;
aggregating the beads using a magnetic field;
removing the binding buffer and the hybridization solution from the aggregated beads;
adding a washing buffer to the beads after the binding buffer and the hybridization solution have been removed, wherein the washing buffer has a temperature and a stringency, and wherein the stringency of the washing buffer is adjusted to provide a melting temperature between the first binding site with the first nucleotide sequence and the second binding site with the second nucleotide sequence of 5 to 10 degrees Celsius above the temperature of the washing buffer;
aggregating the beads to which the washing buffer has been added;
removing the washing buffer from the aggregated beads;
adding an elution buffer to the beads after the washing buffer has been removed;
aggregating the beads to which the elution buffer has been added; and
removing the elution buffer from the aggregated beads, wherein the elution buffer contains purified hybridized RNA polynucleotides from which substantially all DNA oligonucleotides that are not hybridized to RNA polynucleotides have been removed.
